# EUROPEAN PATENT APPLICATION

(11) **EP 1 916 525 A1**
(43) Date of publication of application: **30.04.2008**
(21) Application number: 07250240.4
(22) Date of filing: 22.01.2007
(51) Int. Cl.: G01N 33/566, G01N 33/569

(54) **Method for detection of yeast cell wall**

(30) Priority: 27.10.2006 US 553737
(71) Applicant: Alltech, Inc., Nicholasville, KY 40356 (US)
(72) Inventor: Horgan, Karina, Dublin (IE)
(74) Representative: Robertson, James Alexander

(57) **Abstract**

Methods are provided for detecting the presence of a yeast cell wall in a food or animal feed suspected of containing the yeast cell wall. The method includes the steps of solubilizing the yeast cell wall, adding a lectin which preferentially binds to a mannose component of the solubilized yeast cell wall, detecting the presence of the lectin. In one embodiment, the lectin preferentially binds to an α-linked mannose component of the yeast cell wall. The α-linked mannose component may be an α-(1,3)-linked mannose or an α-(1,6)-linked mannose, and the yeast cell wall may be derived from *Saccharomyces cerevisiae.* Kits for accomplishing the method are described, which include a labeled lectin which preferentially binds to a mannose component of the solubilized yeast cell wall and reagents for detecting the labeled lectin.

## Description

### Technical Field

This invention relates broadly to detection of a yeast cell wall in a food or animal feed. In particular, the invention relates to a lectin-based method for detection and quantification of a yeast cell wall in a food or animal feed. Kits for accomplishing the method are described also.

### Background of the Invention

Various reasons for desiring to detect a yeast cell wall in a food or animal feed exist, as an indicator of the presence of a yeast therein. For example, certain yeasts are indicative of spoilage or contamination of such foods or animal feeds. Early detection of such yeasts or components thereof may provide an "early warning system" for food or animal feed spoilage, preventing or reducing the risk of consumption of such potentially spoiled foods/feeds. A number of methods for non-specifically detecting such yeasts/yeast components are known in the art, ranging in complexity from simple growth on differential media to specific antibody-based assays. Alternatively, it may be desirable to include particular yeasts or yeast components in a food or animal feed. This is because of the growing need for alternatives to traditional growth promoters such as antibiotics in animal feeds.

Animal feeds represent the second largest consumer of antibiotics, following human therapeutic uses. The Union of Concerned Scientists (Union of Concerned Scientists, 2003) estimates that 11.4 million kilograms of antibiotics are used in medicated feeds in the US each year. The discovery that antibiotics improved growth and feed efficiency led to widespread prophylactic antibiotic use (Visek, 1978), which continues in situations where undiagnosed or sub-clinical systemic infections could limit growth and feed efficiency. It has been proposed that the performance benefits from the feeding of antibiotics is due to their ability to inhibit harmful bacteria that adhere to the surfaces of the intestinal villi causing irritation, tissue damage and impeding the absorption of nutrients. The net effect of using antibiotic growth promoters in the poultry industry is estimated to be a 3-5 % increase in the growth and feed conversion efficiency (Thomke and Elwinger, 1998).

However, it has been argued that continued, unregulated, excessive use of antibiotic growth promoters (AGPs) in animal feed imposes a selection pressure for bacteria that are resistant to antibiotics (JETACAR, 1999). The occurrence of vancomycin resistant Enterococci in hospitals, which has been linked to the AGP avoparcin (Collignon, 1999), and the general fears of the development of an antibiotic resistant "super-bacteria" led to the reduction of AGPs registered for use in animal feeds. In 1999, the European Union (EU) placed a partial ban on the use of AGPs which was replaced at the end of 2005 by a general ban of all AGPs. Similarly, countries outside the EU have already started to reduce the levels of antibiotics in all animal feed and it is anticipated that a global ban on the use of AGPs will be implemented in the future.

The Animal Health Institute of America (Animal Health Institute, 1998) has estimated that without the use of AGPs, the USA would require an additional 452 million chickens, 23 million more cattle and 12 million more pigs to reach levels of production attained by the current practices. A total ban on the use of prophylactic and therapeutic antibiotics could cost the US consumers an estimated US$ 1.2-2.5 billion per year (Gill and Best, 1998). As a result producers worldwide are looking for natural alternatives to antibiotics (Mourao, *et al.,* 2006).

The search for alternatives to antibiotics as growth promoters has led to consideration of natural alternatives such as probiotics, prebiotics, organic acids (acidifiers/acidulants), and plant products such as essential oils and oligosaccharides. Similarly, mannose and mannanoligosaccharides have been extensively considered as alternative growth promoters. This is because of the known method by which bacteria, including undesirable bacteria such as enteric pathogens, colonize and infect an organism.

Bacteria possess lectins on their cell surface, which bind to mannose glycoconjugate (glycoprotein and glycolipid) receptors on the surface of mammalian host cells, resulting in attachment and infection. Mannose however, binds competitively to the bacterial lectins, thus occupying sites that would normally bind host cell mannose receptors. This can prevent attachment and without bacterial attachment, infection is prevented (Gardiner, 2000).

However, relatively high concentrations of mannose are required to control colonization of the gut by pathogenic bacteria. Pure mannose is expensive to manufacture and is therefore not used in commercial production units (Spring, *et al.,* 2000). A cheaper source of mannose can be found in plants and bacteria in the form of mannanoligosaccharides, which in certain instances may be even more effective. For example, the complex α-(1,3) and α-(1,6) branched mannan oligosaccharides found in the outer layer of *Saccharomyces cerevisiae* cell wall, are up to 37 times more effective in binding *E.coli* than pure D-mannose (Firon, *et al.,* 1983). While not wishing to be bound by any particular theory, mannanoligosaccharides are believed to have at least three distinct modes of action by which colonisation by pathogenic bacteria is reduced and thus performance in animals is improved. These are: (1) Adsorption of pathogenic bacteria containing Type I fimbriae *(Spring, et al.,* 2000); (2) Improved intestinal function, for example increases in villi height, uniformity and integrity (Loddi, *et al.,* 2002); and (3) Immune modulation stimulating gut-associated and systemic immunity by acting as a non-pathogenic microbial antigen, giving an adjuvant-like effect (Hooge, 2003). A number of yeast cell wall-based commercial products (for example BIO-MOS, MOS 500 and AGRI-MOS, all from Alltech, Inc., Nicholasville, Kentucky, U.S.A.) have therefore been developed and evaluated, and are considered to be safe, effective replacements for potentially undesirable additives such as antibiotics.

It is necessary to quantify any additive in a particular food or animal feed, even a natural additive such as a mannanoligosaccharide. This is due to the need for quality control in a manufactured product, and also to the continuing evolution of legislative and regulatory requirements for the food and animal feed industries. The manufacturer must be able to assure a consistent concentration of the additive, and the consumer must be able to verify the same. Still further, legislative/regulatory bodies, in establishing regulations for particular food/animal feed additives, consistently require a means of reliably detecting and quantifying such additives as part of the consumer protection function such bodies serve. There is accordingly identified a need in the art for a consistent, reliable assay for detecting a mannanoligosaccharide additive in a complex substance such as a food or animal feed composition.

### Summary of the Invention

The present invention seeks to overcome the prior art disadvantages. In one aspect of the present invention a method is provided for detecting the presence of a yeast cell wall in a food or animal feed suspected of containing the yeast cell wall. The skilled artisan will understand that "yeast cell wall" encompasses any portion of the cell wall material of a yeast, and includes intact yeast cell walls as well as yeast cell wall fragments or portions, yeast cell wall derivatives, and the like. It will be appreciated also that the term "animal feed" is intended to include any composition provided to an animal for consumption, such as for example a complete feed, a component intended for incorporation into a complete feed, a nutritional supplement, a premix, or the like. The food or animal feed may be a grain-based animal feed, or any other suitable form in accordance with the consumer's particular dietary requirements.

Thus, according to a first aspect of the present invention there is provided a method for detecting the presence of a yeast cell wall in a food or animal feed suspected of containing the yeast cell wall, comprising:
(i) solubilizing any yeast cell wall in said food or animal feed;
(ii) adding a lectin which preferentially binds to a mannose component of the solubilized yeast cell wall;
(iii) detecting the presence of any lectin-yeast cell wall binding reaction; and
(iv) correlating the results of detection step (iii) with the presence of said yeast cell wall in said food or animal feed.

Further preferable features are described in the appended dependent claims.

Accordingly, the described embodiment relies on the known affinity, or preferential binding, of lectins for components of yeast cell wall. The step of solubilizing the yeast cell wall allows extraction and/or separation of the yeast cell wall from potentially interfering compositions contained in the food or animal feed.

Typically, the lectin preferentially binds to an α-linked mannose component of the yeast cell wall. Suitable lectins include the group of lectins consisting of *Allium ascalonium* agglutinin, *Allium sativum* agglutinin, *Allium ursinium* agglutinin, *Galanthus nivalis* agglutinin, *Hippeastrum hybrid* agglutinin, *Helianthus tuberosus* agglutinin, *Lens culinaris* agglutinin, *Listera ovata* agglutinin, *Narcissus pseudonarcissus* lectin, *Pisum sativum* agglutinin, and combinations thereof. In one embodiment, the lectin preferentially binds to at least one of an α-(1,3)-linked mannose or an α-(1,6)-linked mannose. In this embodiment, the lectin may be at least one of a *Narcissus pseudonarcissus* lectin or a *Galanthus nivalis* agglutinin, and the yeast cell wall may be derived from *Saccharomyces cerevisiae.* The solubilized yeast cell wall may be bound to a solid surface, such as a culture dish, a culture tube, a well of a microtiter plate, or the like, prior to adding the lectin.

Also provided according to the present invention is a kit for detecting the presence of a yeast cell wall in a food or animal feed in accordance with claim 1, comprising:
(i) a labeled lectin which preferentially binds to a mannose component of solubilized yeast cell wall; and
(ii) at least one reagent for detecting the labeled lectin.

Further preferable features are defined in the appended dependent claims.

The reagent is provided to elicit a detectable reaction from the labeled lectin. It will be appreciated that any suitable assay system for detecting the reaction is contemplated, including detection of the reaction by a colorimetric assay, a fluorescent assay, a radioactive assay, a chemiluminescent assay, or any other suitable detection system. The skilled artisan will readily appreciate that the method of the present invention is readily adaptable to a number of known detection systems, such as high performance liquid chromatography, mass spectrometry, chemiluminescence, nuclear magnetic resonance, column separation/column chromatography, lectin affinity chromatography, and the like, with the proviso that the detection system is capable of detecting the labeled lectin.

In one embodiment, the reaction is detected by a colorimetric assay, and the reagent includes a substrate for eliciting a colorimetric reaction from the detector molecule. For example, the lectin may be labeled with biotin, and the reagent may include avidin or streptavidin linked to a peroxidase and a chromogenic substrate which elicits a colorimetric reaction from the peroxidase. However, the present method is not restricted to such a detection system, since other suitable detection methods and assays are contemplated. Accordingly, the kit of the present invention is similarly not to be considered as restricted. For example, the lectin may be labeled with a fluorescent molecule, a chemiluminescent molecule, a radioactive molecule, and the like, and detected using a suitable detector molecule or detector system. For example, a variety of labeling systems are commercially available, including the Fluorescein Protein Labeling Kit and Digoxigenin Protein Labeling Kit (Roche Applied Science, Indianapolis, IN) which can be used to label a detector molecule such as streptavidin. Similarly, commercially available kits exist for labeling molecules such as streptavidin with a radioactive label (for example, from GE Healthcare Life Sciences, Piscataway, NJ). The labeled streptavidin could then be detected using the appropriate detection system. A solvent may be included for solubilizing the yeast cell wall, selected from a group of solvents including, but not limited to, dimethyl sulfoxide, sodium hydroxide, potassium hydroxide, boric acid, acetone, water, and combinations or mixtures thereof. In one embodiment, the solvent is dimethyl sulfoxide.

### Brief Description of the Drawings

The accompanying drawings incorporated in and forming a part of the specification, illustrate several aspects of the present invention, and together with the description serve to explain certain principles of the invention. In the drawings:
Figure 1 schematically depicts an assay for measurement of a yeast cell wall in an animal feed in accordance with the method of the present invention;
Figure 2 shows affinity of several lectins for yeast cell wall;
Figure 3 graphically depicts performance of the assay depicted in Figure 1 on measurement of a yeast cell wall in starter, grower, and finisher pig feed;
Figure 4 graphically depicts performance of the assay depicted in Figure 1 on measurement of a yeast cell wall in pig feed, with measurements taken on five different days; and
Figure 5 graphically depicts performance of the assay depicted in Figure 1 on measurement of a yeast cell wall in poultry feed, with measurements taken on five different days.

Reference will now be made in detail to the present preferred embodiment of the invention, an example of which is illustrated in the accompanying drawings.

### Detailed Description of the Invention

In accordance with the foregoing description, the present invention provides methods and kits for detecting the presence of a yeast cell wall in a food or animal feed suspected of containing the yeast cell wall. In one aspect, the present invention provides a colorimetric method for detecting a yeast cell wall in a food or animal feed, comprising binding a labeled lectin to that yeast cell wall and detecting a colorimetric reaction indicative of the bound lectin. Of course, the skilled artisan will realize that any number of known methods for detecting such binding may be utilized. For example, the lectin could be conjugated with a fluorescent label, a radioactive label, or the like using methods known in the art, and subsequently detected using a suitable detector or reagent system in accordance with the selected label.

In the embodiment described below, a microtiter plate-based assay is described. However, the skilled artisan will readily realize that the method of the present invention is readily adaptable to a variety of assay systems, such as individual sample tubes, or more preferably high through-put systems which automate sample and reagent dispensing, incubation, reaction detection, data acquisition, and analysis. As previously described, the present invention is readily adaptable to a number of known assay systems, such as high performance liquid chromatography, mass spectrometry, chemiluminescence, nuclear magnetic resonance, column separation/column chromatography, lectin affinity chromatography, and the like, with the proviso that the detection system is capable of detecting the labeled lectin. The manufacturers of such systems typically provide detailed instructions and protocols for use of the systems.

In another aspect of the invention, a kit is provided for detecting a yeast cell wall in a food or animal feed, based on the present method. In one embodiment, the kit comprises a labeled lectin which preferentially binds to a mannose component of a solubilized yeast cell wall, and at least one reagent for detecting the labeled lectin. The reagent elicits a detectable reaction from the labeled lectin, which reaction may be detectable by at least one of a colorimetric assay, a fluorescent assay, a chemiluminescent assay, or a radioactive assay. For embodiments of the present method wherein the reaction is detected colorimetrically, the reagent may further include a chromogenic substrate for eliciting a colorimetric reaction from the detector molecule.

In one embodiment, the selected lectin preferentially binds to an α-(1,3)-linked mannose or an α-(1,6)-linked mannose component of the solubilized yeast cell wall, and the detected yeast cell wall is from *Saccharomyces cerevisiae.* In the embodiments described below, the lectin may be at least one of a *Narcissus pseudonarcissus* lectin or a *Galanthus nivalis* agglutinin. Still further, the kit may include a solvent for solubilizing the yeast cell wall, which may be selected from one or more of dimethyl sulfoxide, sodium hydroxide, potassium hydroxide, boric acid, acetone, water, or combinations thereof. Of course, additional reagents and apparatus may optionally be included with the kits, such as suitable containers, buffers, stopping reagents, instructional materials or protocols, and the like.

The examples presented herein are illustrative of one of the modes best suited to carry out the invention. As it will be realized, the invention is capable of other different embodiments and its several details are capable of modification in various, obvious aspects all without departing from the invention. Accordingly, the drawings, descriptions, and examples provided herein will be regarded as illustrative in nature and not as restrictive. All references cited to herein are incorporated into the present disclosure in their entirety by reference unless otherwise specified.

Certain general materials and methods apply generally to the specific examples set forth herein. Most general laboratory chemicals were purchased from Sigma-Aldrich Ireland. (Tallaght, Dublin, Ireland). NUNC Maxisorp 96-well micro-titre plates were obtained from Bio-Sciences Ltd. (Dun Laoghaire, Dublin, Ireland). *Narcissus pseudonarcissus* lectin (NPL), *Galanthus nivalis* agglutinin (GNA) and Wheat Germ Agglutinin were acquired from Vector Labs (Burlingame, CA, USA). Feeds were obtained from W.Connolly and Sons, Red Mills Ltd. (Goresbridge, Kilkenny, Ireland). Yeast cell wall material (Batch no. 511040003) was provided by Alltech, Inc. (Nicholasville, KY, USA).

All feed samples were ground to a fine powder in a commercial blender prior to mixing. Typical inclusion rates for commercial yeast cell wall products range from 0.5 mg g⁻¹ to 2.0 mg g⁻¹ and therefore a standard curve was prepared to reflect this range. Specifically, a yeast cell wall standard curve was prepared having 5 mg g⁻¹, 2.5 mg g⁻¹, 1.25 mg g⁻¹, 0.65 mg g⁻¹, and 0.32 mg g⁻¹ yeast cell wall in pig or poultry feed as described below. Exact quantities of yeast cell wall were weighed and added to 10 g of feed in a 100 mL sterile sample cup, for example 0.05 g YCWM in 10 g feed is equivalent to 5 mg g⁻¹ or 5 kg tonne⁻¹. Samples were shaken at 500 rpm for 1 h to ensure homogenous mixing.

For solubilization or extraction, standards or unknown samples were added to 500 mL of 100 % DMSO and stirred for 1 h. A 2 mL fraction of this extract was placed in an eppendorf and centrifuged at 14,000 rpm for 2 min. A 1 mL aliquot of the supernatant was then added to 500 mL of autoclaved 0.28 M NaCl (coating solution), and mixed thoroughly. Eight 100 µL aliquots of each sample were then plated on micro-titre plates (see below) and incubated for 16 h at 37 °C.

### Example 1

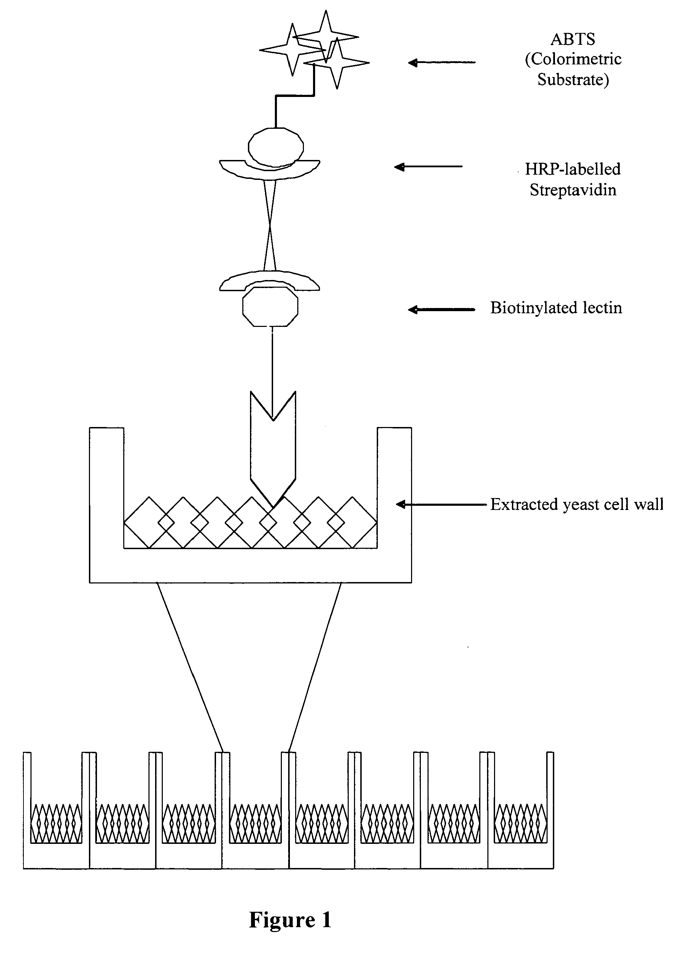

In one embodiment of the present invention, an enzyme-linked lectin assay (ELLA) was developed and used to measure yeast cell wall in feeds. The method is depicted schematically in Figure 1. The present method for the detection and quantification of yeast cell wall material in feed is based on the use of lectins due to their specific saccharide-binding sites. A lectin's affinity for or interaction with certain carbohydrates is as specific as an enzyme-substrate or antigen-antibody interaction. Lectins may bind with free sugars or with sugar residues of polysaccharides, glycoproteins or glycolipids which can be free or bound (Barondes, 1981). Lectins were the preferred choice of affinity ligand in place of antibodies, due to their commercial availability and specificity/sensitivity. A study into the use of lectins in place of antibodies, for the development of an enzyme linked lectin assay (ELLA) for α-(1, 3) galactosyltransferase, showed that lectins produced superior results with respect to both analytical specificity and sensitivity compared to the corresponding antibodies (Khraltsova, *et al.,* 2000).

A number of lectins are commercially available which exhibit enhanced specificity towards particular linkages of the mannose complex of the yeast cell wall (see Table 1). For the depicted embodiment of the present invention, a sensitive, specific assay for cell wall material from *Saccharomyces cerevisiae* was desired. Considering that the mannose segment of *Saccharomyces cerevisiae* cell wall is predominately composed of α-(1, 3) and α-(1,6) mannan linkages, lectins favouring these particular saccharide residues were predominantly considered in selecting lectins for the present invention, to reduce risk of non-specific interference such as from unrelated yeasts or feed constituents with a mannan component. For example, *Candida spp.* produce poly β-(1, 2)-linked mannose chains attached by linkages to their mannans (Gemmil and Trimble, 1999). Of course, it will be appreciated that as desired these other mannans, or indeed other components of yeast cell wall, could be separately or concomitantly detected and/or quantified using a lectin displaying a differing specificity/affinity.

**Table 1: Mannose-binding Lectins**

| | **Source** | **Linkage preference** | **Reference** |
|---|---|---|---|
| **AAA** | Shallot | α-(1, 3) > α-(1, 6) | (Smeets, *et al.,* 1997) |
| *Allium ascalonium* agglutinin | | mannose | |
| | | | |
| **ASA** | Garlic | Mannan | (Kaku, *et al.,* 1992) |
| *Allium sativum* agglutinin | | oligosaccharides | |
| | | | |
| **AUA** | Ramson | α-(1, 3) > α-(1, 6) | (Kaku, *et al.,* 1992) |
| *Allium ursinium* agglutinin | | mannose | |
| | | | |
| **Con A** | Jackbean | α-mannose | (Debray, *et al.,* 1981) |
| Concanavalin A | | α-glucose | |
| | | | |
| **GNA** | Snowdrop | α-(1, 3) mannose | (Shibuya, *et al.,* 1988) |
| *Galanthus nivalis* agglutinin | | | |
| | | | |
| **HHA** | Amaryllis | α-(1, 6) >α -(1, 3) | (Kaku, *et al.,* 1990) |
| *Hippeastrum hybrid* agglutinin | | mannose | |
| | | | |
| **HTA** | Jerusalem | Mannan | (Van *Damme, et al.,* 1999) |
| *Helianthus tuberosus* agglutinin | artichoke | oligosaccharides | |
| | | | |
| **LCA** | Lentil seeds | a-mannose | (Debray, *et al.,* 1981) |
| *Lens culinaris* agglutinin | | α-glucose | |
| | | | |
| **LOA** | Twayblade | Mannan | (Van Damme, *et al.,* 1987) |
| *Listera ovata* agglutinin | | oligosaccharides | |
| | | | |
| **NPL** | Daffodil Bulb | α-(1, 6) mannose | (Kaku, *et al.,* 1990) |
| *Narcissus pseudonarcissus* | | | |
| lectin | | | |
| | | | |
| **PSA** | Peanut | α-mannose | (Debray, *et al.,* 1981) |
| *Pisum sativum* agglutinin | | α-glucose | |

Two lectins *(Narcissus pseudonarcissus* lectin and *Galanthus nivalis* agglutinin) which exhibit enhanced specificity for the mannose linkages present in *Saccharomyces cerevisiae* cell wall material were chosen. Both were obtained commercially available in biotinylated form. Concanavalin A (Con A), a lectin which exhibits a broader specificity for mannose ((α-D-mannose and also α-D-glucose (Debray, *et al.,* 1981)), was also included, and was obtained in horseradish peroxidase (HRP)-labelled form. *Narcissus pseudonarcissus* lectin (NPL) from *Narcissus pseudonarcissus* (Daffodil) bulbs has specificity toward α-linked mannose, preferring polymannose structures containing α-(1, 6) linkages (Kaku, *et al.,* 1990). *Galanthus nivalis* agglutinin (GNA) is isolated from *Galanthus nivalis* (Snowdrop) seeds, and preferentially binds structures containing α-(1, 3) mannose residues, displaying 10-30 times greater affinity for α-(1, 3) linkages compared to D-mannose (Shibuya, *et al.,* 1988).

The suitability of each lectin for the incorporation into an enzyme linked lectin assay (ELLA) for the detection of yeast cell wall material was assessed by analysing their affinity for *Saccharomyces cerevisiae* cell wall material. The yeast cell wall (1.5 µg mL⁻¹) was dissolved in 0.28 M NaCl. This stock solution was serially diluted and eight 100 µl aliquots of each serial dilution were plated on 96-well micro-titre plates and incubated for 16 h at 37 °C. By this step, the yeast cell wall was bound to the microtiter plate well surface. Of course, it will be appreciated that other compositions could be used to achieve the step of binding the yeast cell wall to the microtiter plate surface, such as sodium bicarbonate. Each of the three lectins, either biotinylated (NPL and GNA) or HRP-labelled (Con A), were prepared by diluting the stock lectin solution (2 µg mL⁻¹ lectin) in 10 mM sodium phosphate buffer containing 0.05 % (v/v) Tween 20. The assay was carried out as described below, with the exception that addition of HRP-labeled streptavidin to the Con A samples was unnecessary.

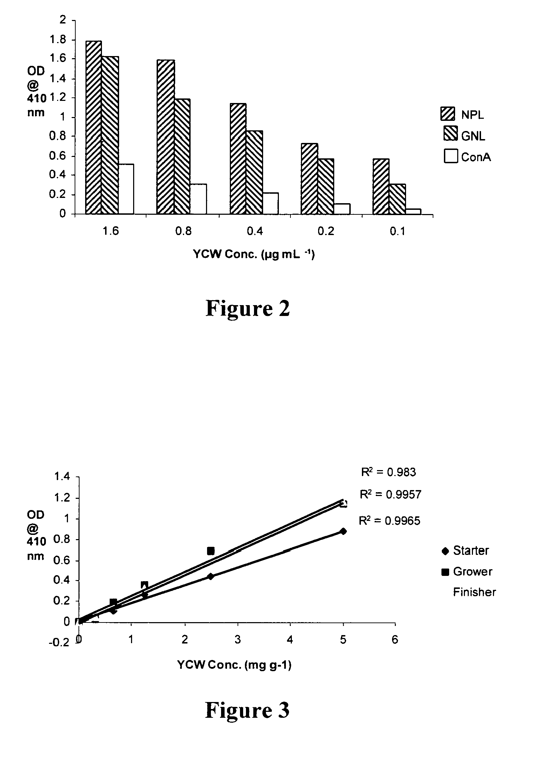

Each lectin displayed some level of affinity for the saccharides present in S cerevisiae yeast cell wall (Figure 2). Absorbance values for Con A were low in comparison with those obtained with NPL and GNA, for example at an inclusion level of 0.4 µg mL⁻¹ the optical density (OD) value obtained using Con A was 0.217 compared to GNA (0.856) and NPL (1.14), indicating that the use of Con A would limit the sensitivity and the detection level of the assay. The use of NPL in the procedure gave slightly higher absorbance levels, at all levels of inclusion, when compared with GNA and was therefore chosen for subsequent experiments.

Standard or unknown sample (100 µl/well), prepared and extracted as described above, was added to the 96-well micro-titre plate and the solution was incubated for 16 h at 37 °C. The micro-titre plate was then emptied and washed three times using a BioTek ELx405 Select CW micro-titre plate washer (BioTek Instruments Inc., Winooski, Vermont, USA) with 300 µL wash buffer (10 mM sodium phosphate buffer pH 7.5). Subsequently, 100 µL of lectin solution (2 µg mL⁻¹ biotinylated *Narcissus pseudonarcissus* lectin in 10 mM sodium phosphate buffer and 0.05 % (v/v) Tween 20) was added to each well and incubated at room temperature for 1 h. The wells were washed (300 µL 10 mM sodium phosphate buffer) three times. Blocking solution ((100 µL of 10 mM sodium phosphate buffer containing 0.1 % (v/v) Tween 20 and 4 % (w/v) BSA)), was added to each well and incubated at room temperature for 1 h to prevent non-specific binding, and the plate was again washed as described above. Each well was then treated with 100 µL Streptavidin Peroxidase HRP-conjugate solution (2.2 µg mL⁻¹ HRP conjugate in sodium phosphate buffer which contained 0.1 % (v/v) Tween 20 and 2 % (w/v) BSA) and the plate was incubated at room temperature for 1 h.

After three additional post-incubation washes, 100 µL of substrate (2, 2'-Azino-bis 3-ethylbenzthiazoline-6-sulfonic acid) was added to each well. The reaction was stopped, following incubation at room temperature for 20 min, by the addition of stopping reagent (100 µL 0.5 M oxalic acid) to each well. Optical density (OD) of the resulting sample was read at 410 nm, using a BioTek Synergy HT micro-titre plate reader (BioTek Instruments Inc.). Of course, the skilled artisan will appreciate that the specific OD setting was selected according to the microtiter plate reaction vessel selected, and will vary in accordance with the particular reaction vessel used. Procedures for optimisation of such assay parameters are well within the knowledge of the skilled artisan. A standard curve was generated by plotting absorbance of each standard against concentration of that standard. The standard curve was used to quantify yeast cell wall content of unknown feed samples.

### Example 2

It was desired to verify that the method of the present invention was effective in detecting/quantifying yeast cell wall in varying "sub-types" of animal feeds. Specifically, three general sub-types of animal feeds are recognized for most domesticated animals, i.e., starter feeds, grower feeds, and finisher feeds. These feed sub-types vary in their constituents, i.e., protein, fiber, ash, trace minerals, vitamins, etc. in accordance with the particular animal's needs at the particular stage of growth/development. A yeast cell wall standard curve, prepared as described above, was prepared for each of the three sub-types of both pig and poultry feed to assess the efficacy of the present method over a range of feed types.

Despite differences in the formulations for each pig feed sub-type (the protein content of the feed varies from 17.5 % for finisher feed to 18.0 % for grower feed and 21 % for starter feed, based on the nutritional requirements of the animal at each particular stage of their growth), the difference in standard curves obtained (Figure 3) was not substantial, i.e. the absorbance values at any one inclusion level differed by a maximum of 0.26 OD. At an inclusion level of 5 mg g -1, the OD readings for grower (1.14) and starter (1.16) feeds were very similar and those obtained for starter (0.90) were only slightly lower. The extraction method and the ELLA procedure, therefore, proved compatible with all sub-types of pig feed. Similarly, despite the variation in composition of poultry feed sub-types (ash content varied from 13 % for finisher feed to 5 % for grower and 6 % for starter), only minor differences were observed in the standard curves, i.e. a maximum difference of 0.24 OD at any one inclusion level (data not shown). At an inclusion level of 5 mg g⁻¹, OD values for starter (1.158) and grower (1.153) were almost identical and those obtained for finisher (0.92) were only slightly lower. As such, the method of the present invention was also compatible with all sub-types of poultry feed.

### Example 3

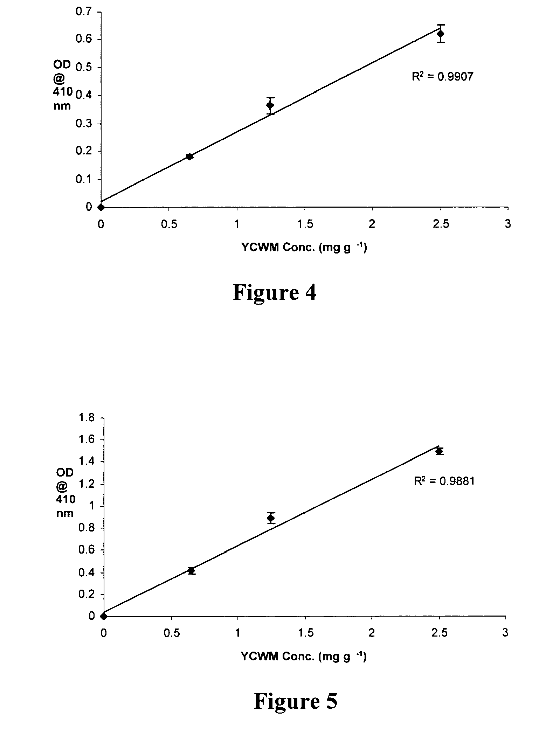

To test the reliability of the assay, a yeast cell wall standard curve was constructed as described above in both pig (finisher) and poultry (finisher) feeds on each of five different days. The mean, standard deviation (SD), standard error of the mean (SEM) and the p-value of the absorbance values obtained, on each of the five days, at each inclusion level were then calculated. A one way ANOVA statistical analysis was carried out on the data obtained to determine if there was any significant variance between the results from day to day. Each inclusion level of the standard curve was compared for each of the five days. Results are shown below for pig feed (Figure 4) and poultry feed (Figure 5).

No significant variation was found in the assay over the five day period, at all inclusion levels. This served to demonstrate the reproducibility and reliability of the method to produce similar results from day to day.

### Example 4

To assess specificity, two microtiter plates were coated, one with yeast cell wall material (10 µg) and the second with N-acetyl glucosamine (10 µg). Each plate was assayed as described above. In a second experiment two plates were coated in the same manner and assayed using the present method but replacing the *Narcissus pseudonarcissus* lectin with a wheat germ agglutinin (WGA), which is specific for N-acetyl glucosamine dimers and trimers. In addition, yeast cell wall material was spiked into pig feed and poultry feed, at an inclusion level of 5 mg g⁻¹ and these were assayed with both the *Narcissus pseudonarcissus* lectin and the wheat germ agglutinin (WGA). This served to investigate if components of the feed would impact negatively on results due to a large degree of background interference. All experiments were carried out in duplicate. Results are shown in Table 2.

**Table 2: Specificity of Narcissus pseudonarcissus lectin for yeast cell wall material.**

| | **Treatment** | **OD _{410 nm} Value** |
|---|---|---|
| **yeast cell wall** | Wheat Germ | 0.011 |
| (0.2 µg mL⁻¹) | Agglutinin | |
| | *N.pseudonarcissus* | 1.358 |
| | lectin | |
| **Poultry Feed + yeast** | Wheat Germ | 0 |
| **cell wall** | Agglutinin | |
| (0.2 µg mL⁻¹) | | |
| | *N.pseudonarcissus* | 0.775 |
| | lectin | |
| **Pig Feed+ yeast cell** | Wheat Germ | 0 |
| **wall** | Agglutinin | |
| (0.2 µg mL⁻¹) | | |
| | *N.pseudonarcissus* | 1.025 |
| | lectin | |
| **N-Acetyl glucosamine** | Wheat Germ | 1.947 |
| (0.2 µg mL⁻¹) | Agglutinin | |
| | *N.pseudonarcissus* | 0 |
| | lectin | |

| | | |
|---|---|---|
| All standards were extracted in duplicate. Each value is expressed as a mean of sixteen OD ₄₁₀ₙₘ absorbance measurements. | | |

The use of NPL in the present method produced a colorimetric response for yeast cell wall (1.358 @ OD₄₁₀ₙₘ) and for yeast cell wall extracted from both pig (1.025) and poultry feed (0.775). In contrast to this, no colorimetric response was obtained when N-Acetyl glucosamine was used as a substrate for NPL further demonstrating its specificity for α-(1, 3) and α-(1, 6) mannose residues of the yeast cell wall.

In addition to this, little or no colorimetric response was observed when NPL was replaced with WGA. This demonstrates the lack of affinity of WGA for yeast cell wall and yeast cell wall extracted from feed. Based on the results obtained, it has been shown that the present method can measure, with suitable specificity, yeast cell wall in the presence or absence of feed.

### Example 5

As a portion of the validation procedure, the limit of detection (LOD) and limit of quantitation (LOQ) of the present method were established. The LOD of an individual analytical procedure is the lowest amount of analyte in a sample which can be detected but not necessarily quantitated as an exact value (ICH, 1995). The limit of quantification of an individual analytical procedure is the lowest amount of analyte in a sample which can be quantitatively determined with suitable precision and accuracy.

If a LOD determination is to be based on the standard deviation of the blank response and the slope of standard curve then, the following equation applies:
LOD = 3.3 σ/S, where σ= Standard deviation of the response and S = Slope of the standard curve.
   If a LOQ determination is to be based on the standard deviation of the blank response and the slope of standard curve then, the following equation applies:
LOQ = 10 σ/S, where σ = Standard deviation of the response and S = Slope of the standard curve.

Ten blank feed samples were assayed and the mean and standard deviation between samples were calculated for each feed type. A standard curve for both pig and poultry feed was also generated as described above, and the slope of the line noted. Results obtained are shown in Table 3.

**Table 3: Determination of LOD and LOQ for detection of yeast cell wall in pig and poultry feed.**

| | **Poultry Feed** | **Pig Feed** |
|---|---|---|
| **Mean** | 0.1660 | 0.3558 |
| **Standard Deviation** | 0.0160 | 0.0095 |
| **(σ)** | | |
| **Slope of Standard** | 0.3691 | 0.2727 |
| **Curve (S)** | (Figure 3.13) | (Figure 3.12) |
| **Limit of Detection** | (3.3* | (3.3* |
| **(LOD) = (3.3σ/S)** | 0.0160)/0.3691 | 0.0095)/0.2727 |
| | 0.14 mg g⁻¹ | 0.12 mg g⁻¹ |
| **Limit of** | (10*0.0160)/0.3691 | (10* |
| **Quantification** | 0.45 mg g⁻¹ | 0.0095)/0.2727 |
| **(LOQ) = (10σ/S)** | | 0.35 mg g⁻¹ |

| | | |
|---|---|---|
| n = 10. Each value is expressed as a mean of eight OD ₄₁₀ₙₘ absorbance measurements. | | |

The limit of detection for poultry feed was determined to be 0.14 mg g⁻¹ and the limit of quantification was determined to be 0.45 mg g⁻¹. The limit of detection for pig feed was determined to be 0.12 mg g⁻¹ and the limit of quantification was determined to be 0.35 mg g⁻¹. The observed difference in LOD and LOQ for pig and poultry feed is most likely due to a lack of homogeneity of constituents in the poultry feed used, which resulted in a higher standard deviation of the blanks (0.0160 for poultry feed compared with 0.0095 for pig feed). The higher standard deviation of the blanks for poultry feed resulted in higher limits of detection and quantification for poultry feed.

While the LOQ differs between feed types, in both cases the LOQ is below the typical minimal inclusion level of yeast cell wall-based commercial products such as BIO-MOS (Alltech Inc.) used in poultry and pig applications (typically 0.5 mg g⁻¹). This confirms that the assay can reliably quantify yeast cell wall in poultry and pig feed at and below typical levels of inclusion currently advised.

### Example 6

Accuracy was assessed at a typical level of inclusion (1.0 mg g⁻¹) and also at the defined limit of quantification for both pig (0.35 mg g⁻¹) and poultry (0.45 mg g⁻¹) feed. Accuracy is expressed as percent accuracy: mean estimated unitage/assigned unitage x 100. Ten individual samples for each inclusion level and feed type to be tested were prepared, extracted, and analyzed as described above. The percentage accuracy of each was then calculated. Results for both pig feed (Table 4) and poultry feed (Table 5) are shown below.

**Table 4: Accuracy of quantification of yeast cell wall material in pig feed.**

| **Pig Feed** | **Accuracy @ Typical Level of Inclusion** | **Accuracy @ LOQ** |
|---|---|---|
| | **(1.0 mg g⁻¹⁾** | **(0.35 mg g⁻¹)** |
| **Mean Estimated** | 1.03 ± 0.030 | 0.34 ± 0.028 |
| **Conc.(mg g⁻¹)** | | |
| **Actual Conc. (mg g⁻¹)** | 1.0 | 0.35 |
| **% Accuracy** | 97.52 | 97.6 |

| | | |
|---|---|---|
| Values are expressed as the mean of 10 individual estimations of yeast cell wall material content ± standard deviation. Each estimate is based on a mean of eight OD ₄₁₀ₙₘ absorbance measurements. | | |

**Table 5: Accuracy of quantification of yeast cell wall material in poultry feed.**

| **Poultry Feed** | **Accuracy @ Typical** | **Accuracy @ LOQ** |
|---|---|---|
| | **Level of Inclusion** | **(0.45 mg g⁻¹)** |
| | **(1.0 mg g⁻¹)** | |
| **Mean Estimated** | 1.03 ± 0.06 | 0.47 ± 0.04 |
| **Conc.(mg g⁻¹)** | | |
| **Actual Conc. (mg g⁻¹)** | 1.0 | 0.45 |
| **% Accuracy** | 97.1 | 96.4 |

| | | |
|---|---|---|
| Values are expressed as the mean of 10 individual estimations of yeast cell wall material content ± standard deviation. Each estimate is based on a mean of eight OD_{410 nm} absorbance measurements. | | |

It is recommended by the FDA (FDA, 2005) that the accuracy obtained using fortified blank matrix samples should be between 80-110 %. All accuracy studies undertaken were within this range. It is therefore accepted that the assay is accurate over a range of values for both poultry and pig feed. Often a decreased level of accuracy is observed as concentration of analyte, i.e. yeast cell wall material, decreases. However, this was not evident in terms of the present method, and a consistent level of accuracy was maintained over all inclusion levels investigated.

### Example 7

For single-laboratory validation, two sets of conditions are relevant: (a) precision under repeatability conditions, describing variations observed during a single run, and (b) precision under run-to-run conditions (Thompson*, et al.,* 2002). Precision under repeatability conditions was estimated as follows. A sample of known yeast cell wall concentration (1.0 mg g⁻¹) was extracted as described above and ten 1 mL aliquots from the extract retained. Each aliquot was assayed as described above. The mean and the standard deviation of the 10 OD values obtained were calculated and results were expressed as RSD = (SD/mean x 100). The precision under run-to-run conditions was estimated as follows. Ten separate samples having the same yeast cell wall concentration (1.0 mg g⁻¹) were individually extracted as described above. Each sample was assayed as described above. The mean and standard deviation of OD values obtained for the ten replicates were calculated and results were expressed as RSD = (SD/mean x 100).

The precision of estimation of yeast cell wall content was also investigated at a typical level of inclusion and at the LOQ. A typical standard curve, prepared as described above, was constructed. Ten replicates for each inclusion level (LOQ and 1.0 mg g⁻¹ for each feed type) to be tested were prepared as described above, and extracted as outlined above. All samples were analyzed and the yeast cell wall content calculated based on the equation of the line of the standard curve obtained. The estimated concentrations were compared to assess the level of precision of the assay at the respective concentrations. The mean and standard deviation for each inclusion level tested was calculated and the results were expressed as RSD = (SD/mean x 100). The results for all precision investigations, obtained for pig feed (Table 6) and poultry feed (Table 7) are shown below.

**Table 6: Precision of quantification of yeast cell wall in pig feed.**

| | **Precision** | **Precision** | **Precision of** | **Precision of** |
|---|---|---|---|---|
| | **(run to** | **(Repeatability)** | **Estimation** | **Estimation** |
| | **run)** | **@ 1.0 mg g** ⁻¹ | **@** | **@ LOQ** |
| | **@ 1.0 mg** | **OD 410 nm** | **Inclusion** | **(0.45 mg g⁻¹)** |
| | **g⁻¹** | | **rate** | |
| | **OD ₄₁₀ₙₘ** | | **(1.0 mg g⁻¹)** | |
| **Mean** | 0.438 | 0.307 | 1.02 | 0.34 |
| **SD** | 0.0405 | 0.028 | 0.11 | 0.03 |
| **RSD** | 9.26 % | 9.25 % | 11.13 % | 8.36 % |
| **(%)** | | | | |

| | | | | |
|---|---|---|---|---|
| All values are expressed as a mean of 10 individual determinations. SD denotes standard deviation. RSD denotes relative standard deviation. | | | | |

**Table 7: Precision of quantification of yeast cell wall in poultry feed.**

| | **Precision** | **Precision** | **Precision of** | **Precision of** |
|---|---|---|---|---|
| | **(run to** | **(Repeatability)** | **Estimation** | **Estimation** |
| | **run)** | **@ 1.0 mg g**^{**-**1} | **@ Inclusion** | **@ LOQ** |
| | **@ 1.0 mg** | **OD _{410 nm}** | **rate** | **(0.45 mg g⁻¹)** |
| | **g-¹** | | **(1.0 mg g⁻¹)** | |
| | **OD _{410 nm}** | | | |
| **Mean** | 1.02 | 0.929 | 1.03 | 0.47 |
| **SD** | 0.075 | 0.028 | 0.068 | 0.041 |
| **RSD (%)** | 7.35 % | 3.05 % | 6.61 % | 8.9 % |

| | | | | |
|---|---|---|---|---|
| All values are expressed as a mean of 10 individual determinations. SD denotes standard deviation. RSD denotes relative standard deviation. | | | | |

The acceptance criteria for precision depend very much on the type of analysis. For instance, the pharmaceutical industry regularly achieves better than 1 % RSD for compound analysis, whereas for biological samples the precision is more likely to be 15 % at the concentration limits and 10 % at other concentration levels. For environmental and food samples, the precision is very much dependant on the sample matrix, the concentration of the analyte and on the analysis technique. It can vary between 2 and 20 %. Prior to validation, an RSD of 15 % was deemed acceptable for this method based on the nature of the matrix, i.e. animal feed (Ludwig, 2000). Each individual precision investigation for both feed types obtained results well under this desired RSD. The results achieved for precision of estimation investigations demonstrates that the present method measures the yeast cell wall content of a feed sample with an acceptable level of precision and accuracy further strengthening confidence in the method for its intended purpose.

### Example 8

The performance of the present method was tested on commercial feed samples. A number of commercial feed samples, ranging in feed type, country of origin, inclusion level and form, were received for analysis. All samples were extracted and analyzed as described above. A yeast cell wall standard curve was prepared as described above using the appropriate blank feed type and yeast cell wall material. The quantity of yeast cell wall in the unknown sample, for each feed type, was then estimated based on the equation of the standard curve. Results are shown in Table 8.

**Table 8: Estimation of yeast cell wall material in commercial feed samples.**

| **No.** | **Origin** | **Feed Type** | **Form** | **Actual** | **Estimated** | **Accuracy** |
|---|---|---|---|---|---|---|
| 1 | Ireland | Pig Starter | Pellet | 1.0 kg | 0.96 kg tonne⁻¹ | 96% |
| | | | | tonne⁻¹ | | |
| 2 | Holland | Breeder | Grain | 1.0 kg | 1.07 kg tonne⁻¹ | 93% |
| | | | | tonne⁻¹ | | |
| 3 | Holland | Broiler | Grain | 1.5 kg | 1.7 kg tonne⁻¹ | 87% |
| | | | | tonne⁻¹ | | |
| 4 | Romania | Broiler Starter | Grain | 2.0 kg | 2.08 kg tonne⁻¹ | 95.9% |
| | | | | tonne⁻¹ | | |
| 5 | Sweden | Broiler Starter | Pellet | 2.0 kg | 1.87 kg tonne⁻¹ | 93.6% |
| | | | | tonne⁻¹ | | |
| 6 | Sweden | Sow Lactation | Pellet | 3.0 kg | | 82.6% |
| | | | | tonne⁻¹ | 2.48 kg tonne⁻¹ | |
| 7 | Sweden | Sow | Pellet | 3.0 kg | | 83.9% |
| | | Gestation | | tonne⁻¹ | 2.52 kg tonne⁻¹ | |
| 8 | Prague | Not Known | Pellet | 2.0 kg | 1.78 kg tonne⁻¹ | 89.1% |
| | | | | tonne⁻¹ | | |
| 9 | Prague | Not Known | Pellet | 1.0 kg | | |
| | | | | tonne⁻¹ | 0.86 kg tonne⁻¹ | 86.1% |

| | | | | | | |
|---|---|---|---|---|---|---|
| All samples were extracted in duplicate. Each estimate is based on a mean of eight OD _{410 nm} absorbance measurements. | | | | | | |

The results obtained indicated that the present method could accurately detect and quantify yeast cell wall in commercial feed samples. The accuracy obtained for all samples was within acceptable levels (i.e. 80 - 110 %, (FDA, 2005)).

### Example 9

The assay described above (see Example 1) is repeated, with the exception that a fluorescein-labeled streptavidin is used to detect the biotinylated lectin. In accordance with the manufacturer's instructions (Roche Applied Science, Cat. No. 11 386 093 001, June 2005), streptavidin is dissolved in phosphate-buffered saline and reacted with a fluorescein reagent supplied by the manufacturer, at a ratio of from about 1:5 to about 1:110 (streptavidin:fluorescein) with stirring. Non-reacted fluorescein is removed by column chromatography on Sephadex G-25. Labeling yield is calculated by measuring extinction at 280 nm (protein) and 495 (fluorescein) using a photometer. The fluorescein-labeled streptavidin is then used to detect the biotin-labeled lectin as previously described, using an appropriate detection system.

### Example 10

The assay described above (see Example 1 ) is repeated, with the exception that a digoxigenin-labeled streptavidin is used to detect the biotinylated lectin. In accordance with the manufacturer's instructions (Roche Applied Science, Cat. No. 11 367 200 001, Nov. 2004), streptavidin is dissolved in phosphate-buffered saline and reacted with a digoxigenin reagent supplied by the manufacturer, at a ratio of from about 1:5 to about 1:110 (streptavidin:digoxigenin) with stirring. Non-reacted digoxigenin is removed by column chromatography on Sephadex G-25. Labeled streptavidin is collected by retaining the first pools of eluate (the first 4 pools of 0.5 ml each). Presence of suitable labeled digoxigenin is verified by measuring extinction at 280 nm with a photometer. The digoxigenin-labeled streptavidin is then used to detect the biotin-labeled lectin as previously described, using an appropriate detection system.

It is accordingly shown that the present invention provides a method capable of accurately, precisely and reliably detecting and quantifying yeast cell wall in animal feeds. The present method accurately quantifies the amount of yeast cell wall-based product in feed samples, and can confidently be applied to commercial samples for the routine detection and quantification of yeast cell wall in animal feed.

The foregoing description of the preferred embodiment of this invention has been presented for purposes of illustration and description. It is not intended to be exhaustive or to limit the invention to the precise form disclosed. Obvious modifications or variations are possible in light of the above teachings. The embodiment was chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

### Citations of Literature

Animal Health Institute (1998). Antibiotic resistance back in the news. AHI Quarterly 19, 1-4.
Collignon, P. J. (1999). Vancomycin-resistant Enterococci and the use of avoparcin in animal feed, is there a link? Medical Journal of Australia 171, 144-146.
Debray, H., Decout, D., Strecker, G., Spik, G. and Montreuil, J. (1981). Specificity of twelve lectins towards oligosaccharides and glycopeptides related to N-glycosylproteins. European Journal of Biochemistry 117, 41-55.
FDA (2005). Guidance for Industry: Validation of Analytical Procedures for Type C Medicated Feeds, Food and Drug Administration. Available Online at (www.fda.gov/cvmlGuidance/guide/135.pdf)
Firon, N., Ofek, I. and Sharon, N. (1983). Carbohydrate specificity of the surface lectins of Escherichia coli, Klebsiella pneumoniae, and Salmonella typhimurium,. Carbohydrate Research 120, 235-249.
Gardiner, T. (2000). Dietary mannose: Absorption, distribution, metabolism, excretion (ADME) and biological activity. Glycoscience and Nutrition 1*.*
Gill, C. and Best, P. (1998). Antibiotic resistance in USA:Scientists to look more closely. Feed Int., 16-18*.*
Hooge, D. M. (2003). Broiler chicken performance may improve with MOS. Feedstuffs 75, 1-5.
JETACAR (1999). The use of antibiotics in food producing animals: antibioticresistant bacteria in animals and humans. Available Online at (http://www.health.gov.au/internet/wcms/Publishing.nsf/Content/health-publicat.htm*).*
Kaku, H., Van Damme, E. J. M., Peumans, W. J. and Goldstein, I. J. (1990). Carbohydrate-binding specificity of the daffodil (Narcissus psuedonarcissus) and amaryllis (Hippeastrum hybr.) bulb lectins. Archives of Biochemistry and Biophysics 279, 298-304.
Khraltsova, L. B., Sablina, M. A., Melikhova, T. D., Joziasse, D. H., Kaltner, H., Gabius, H. J. and Bovin, N. V. (2000). An Enzyme-Linked Lectin Assay for [alpha]-1, 3 galactosyltransferase. Analytical Biochemistry 280, 250-257.
Loddi, M. M., Nakaghi, L. S. O., Edens, F., Tucci, F. M., Hannas, M. I., Moraes, V. M. B. and Ariki, J. (2002). Mannanoligosaccharides and organic acids on intestinal morphology and integrity of broilers evaluated by scanning electron microscopy. In: Proc. 11th European Poult Sci., Conf Bremen, Germany., Sept 6-10. pp121.
Ludwig, H. (2000). Method Validation, Available Online at (www.labcompliance.com/methods/meth val.htm*.).*
Mourao, J. L., Pinheiro, V., Alves, A., Guedes, C. M., Pinto, L., Saavedra, M. J., Spring, P. and Kocher, A. (2006). Effect of mannan oligosaccharides on the performance, intestinal morphology and cecal fermentation of fattening rabbits. Animal Feed Science and Technology 126, 107-120.
Shibuya, N., Goldstein, 1. J., Van Damme, E. J. M. and Peumans, W. J. (1988). Binding properties of a mannose-specific lectin from the snowdrop (Galanthus nivalis) bulb. Journal of Biological Chemistry 263, 728-734.
Spring, P., Wenk, C., Dawson, K. A. and Newman, K. E. (2000). The effects of dietary mannan oligosaccharide on cecal parameters and the concentrations of enteric bacteria in the ceca of Salmonella challenged broiler chicks. Poultry Science 79, 205-211.
Thomke, S. and Elwinger, K. (1998). Growth promotants in feeding pigs and poultry.11. Mode of action of antibiotic growth promotants. Annales de Zootechnie 47, 153-167.
Thompson, M., Ellison, S. L. and Wood, R. (2002). Harmonized guidelines for single-laboratory validation of methods of analysis. Pure Applied Chemistry 74, 835-855.
Union of Concerned Scientists (2003). Antibiotic Resistance. Available Online at (www.ucsusa.org/food_and_environment/*).*
Visek, W. J. (1978). The mode of growth promotion by antibiotics. Journal of Animal Science 46, 1447-1469.

## Claims

1. A method for detecting the presence of a yeast cell wall in a food or animal feed suspected of containing the yeast cell wall, comprising:
(i) solubilizing any yeast cell wall in said food or animal feed;
(ii) adding a lectin which preferentially binds to a mannose component of the solubilized yeast cell wall;
(iii) detecting the presence of any lectin-yeast cell wall binding reaction; and
(iv) correlating the results of detection step (iii) with the presence of said yeast cell wall in said food or animal feed.

2. The method of claim 1, wherein said food or animal feed is a grain-based animal feed.

3. The method according to any preceding claim, wherein said lectin preferentially binds to an α-linked mannose component of the yeast cell wall.

4. The method according to any preceding claim, wherein the lectin preferentially binds to at least one of an α-(1,3)-linked mannose or an α-(1,6)-linked mannose.

5. The method according to any preceding claim, wherein the lectin is selected from the group of lectins consisting of *Allium ascalonium* agglutinin, *Allium sativum* agglutinin, *Allium ursinium* agglutinin, *Galanthus nivalis* agglutinin, *Hippeastrum hybrid* agglutinin, *Helianthus tuberosus* agglutinin, *Lens culinaris* agglutinin, *Listera ovata* agglutinin, *Narcissus pseudonarcissus* lectin, *Pisum sativum* agglutinin, and combinations thereof.

6. The method according to any preceding claim, wherein the lectin is at least one of a *Narcissus pseudonarcissus* lectin or a *Galanthus nivalis* agglutinin.

7. The method according to any preceding claim, wherein the solubilized yeast cell wall is bound to a solid surface prior to adding the lectin.

8. The method according to claim 7, wherein the solubilized yeast cell wall is bound to the solid surface using at least one of sodium chloride or sodium bicarbonate.

9. The method according to any preceding claim, wherein the yeast cell wall is derived from *Saccharomyces cerevisiae.*

10. The method according to any of the preceding claims, said lectin being labeled.

11. The method according to claim 10, additionally comprising the step of adding a reagent for detecting said labeled lectin.

12. The method according to claim 11, wherein said reagent elicits a detectable reaction from said labeled lectin.

13. The method according to claim 12, wherein said reagent further includes a substrate for eliciting a colorimetric reaction from a detector molecule.

14. The method according to claim 13, wherein said lectin is labeled with biotin, and said reagent comprises avidin or streptavidin linked to a peroxidase and a chromogenic substrate which elicits a colorimetric reaction from the peroxidase.

15. The method according to any of the preceding claims, wherein said detection step (iii) comprises a colorimetric assay, a fluorescent assay, a chemiluminescent assay, or a radioactive assay.

16. The method according to any of the preceding claims, said solubilizing step comprising mixing said food or animal feed with a yeast cell wall solvent.

17. The method according to claim 16, said solvent being selected from the group consisting of dimethyl sulfoxide, sodium hydroxide, potassium hydroxide, boric acid, acetone, water, and combinations thereof.

18. A kit for detecting the presence of a yeast cell wall in a food or animal feed in accordance with claim 1, comprising:
(i) a labeled lectin which preferentially binds to a mannose component of solubilized yeast cell wall; and
(ii) at least one reagent for detecting the labeled lectin.

19. The kit of claim 18, wherein the reagent elicits a detectable reaction from the labeled lectin.

20. The kit of claim 19, wherein the lectin preferentially binds to an α-(1,3)-linked mannose or an α-(1,6)-linked mannose component of the solubilized yeast cell wall.

21. The kit of claim 19, wherein the reaction is detectable by at least one of a colorimetric assay, a fluorescent assay, a chemiluminescent assay, or a radioactive assay.

22. The kit of claim 21, wherein the reaction is detectable by a colorimetric assay.

23. The kit of claim 19, wherein the reagent further includes a substrate for eliciting a colorimetric reaction from a detector molecule.

24. The kit of claim 23, wherein the lectin is labeled with biotin, and the reagent comprises avidin or streptavidin linked to a peroxidase and a chromogenic substrate which elicits a colorimetric reaction from the peroxidase.

25. The kit of claim 18, further including a solvent for solubilizing yeast cell wall.

26. The kit of claim 25, wherein the solvent is selected from the group of solvents consisting of dimethyl sulfoxide, sodium hydroxide, potassium hydroxide, boric acid, acetone, water, and combinations thereof.

27. The kit of claim 26, wherein the solvent comprises dimethyl sulfoxide.

28. The kit of claim 20, wherein the lectin is at least one of a *Narcissus pseudonarcissus* lectin or a *Galanthus nivalis* agglutinin.
